(19) Europäisches Patentamt
European Patent Office
Office européen des brevets

(11) EP 0 857 083 B1

(12) # EUROPÄISCHE PATENTSCHRIFT

(45) Veröffentlichungstag und Bekanntmachung des Hinweises auf die Patenterteilung:
**14.06.2000 Patentblatt 2000/24**

(21) Anmeldenummer: **96934716.0**

(22) Anmeldetag: **17.10.1996**

(51) Int Cl.[7]: **B01J 31/40**, B01J 31/02, C07D 307/28

(86) Internationale Anmeldenummer:
**PCT/EP96/04504**

(87) Internationale Veröffentlichungsnummer:
**WO 97/15392 (01.05.1997 Gazette 1997/19)**

(54) **VERFAHREN ZUR ABTRENNUNG VON OLIGOMEREN NEBENPRODUKTEN VON EINEM ISOMERISIERUNGSKATALYSATOR**

METHOD OF SEPARATING OLIGOMERIC SIDE-PRODUCTS FROM AN ISOMERIZATION CATALYST

PROCEDE DE SEPARATION DE SOUS-PRODUITS OLIGOMERES D'UN CATALYSEUR D'ISOMERISATION

(84) Benannte Vertragsstaaten:
**AT BE DE DK ES FI FR GB GR IE IT NL SE**

(30) Priorität: **23.10.1995 DE 19539331**

(43) Veröffentlichungstag der Anmeldung:
**12.08.1998 Patentblatt 1998/33**

(73) Patentinhaber: **BASF AKTIENGESELLSCHAFT
67056 Ludwigshafen (DE)**

(72) Erfinder: **FISCHER, Martin
D-67071 Ludwigshafen (DE)**

(56) Entgegenhaltungen:
**EP-A- 0 412 366**
**WO-A-94/23837**
**US-A- 5 238 889**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 857 083 B1

Printed by Jouve, 75001 PARIS (FR)

## Beschreibung

Die Erfindung betrifft ein Verfahren zur Abtrennung von Oligomeren des Vinyloxirans von einem Isomerisierungskatalysator, zum Zwecke der Wiedergewinnung des Katalysators für die Isomerisierung von Vinyloxiran zu 2,5-Dihydrofuran, bestehend im wesentlichen aus einem Oniumiodid, Zink-, Zinn-, Kobalt-, oder Bismuthhalogenid und gegebenenfalls einem Donorliganden, wobei der mit Oligomeren verunreinigte Katalysator mit einem unpolaren Kohlenwasserstoff- oder Chlorkohlenwasserstoff-Extraktionsmittel vermischt wird, die sich dabei bildenden Phasen getrennt werden und das Extraktionsmittel von der den Katalysator enthaltenden Phase abdestilliert wird. Der Katalysator kann dann wieder in die Isomerisierungsreaktion zurückgeführt werden.

Die Isomerisierung von Vinyloxiran zu 2,5-Dihydrofuran ist in zahlreichen Patentschriften, z.B. in US 5,082,956, EP 0412366, WO 93/10111 und DE-A 44 24 219 (O.Z. 45023) beschrieben.
Bei der Isomerisierung entstehen oligomere Nebenprodukte, die sich im Reaktionsgemisch anreichern, wenn das Produkt Dihydrofuran fortlaufend abgetrennt wird. Es ist deshalb erforderlich, einen Teil des Katalysator/Oligomerengemisches auszuschleusen, um eine bestimmte Höchstkonzentration von Oligomeren in dem Reaktionsgemisch nicht zu überschreiten. Da der ausgeschleuste Katalysator aus Kostengründen nicht verworfen werden kann, muß er aus dem ausgeschleusten Gemisch in einer Form wiedergewonnen werden, die es erlaubt, ihn ohne Aktivitätsverlust in die Isomerisierungsphase zurückzuführen.

Zu diesem Ziel beschreibt US 5 238 889 die Abtrennung von oligomeren Nebenprodukten, die bei der mit Tetraalkylphosphoniumiodiden und Triphenyl- oder Tri-n-octyl-zinniodiden katalysierten Isomerisierung von Vinyloxiran zu 2,5-Dihydrofuran gebildet werden, durch Zusatz eines unpolaren Lösungsmittels, Abtrennung des in dem Lösungsmittel ungelösten Anteils der oligomeren Nebenprodukte als zweite Phase und destillative Entfernung des Lösungsmittels.

Von Nachteil für die Durchführung der in US 5 238 889 beschriebenen Kombination von Isomerisierung und Nebenproduktabtrennung in technischem Maßstab ist der Einsatz der teuren, in großem Maßstab nicht verfügbaren Organozinniodide als Katalysatorkomponenten.

Erschwerend kommt noch hinzu, daß Organozinnhalogenide durch Wasser oder andere protische Reaktionspartner leicht zersetzt werden. So besteht die Gefahr, daß Spuren Wasser in dem für die Isomerisierung eingesetzten Vinyloxiran zu einer Desaktivierung des Katalysators durch die Reaktionsfolge

$$R_3SnJ \xrightarrow{H_2O} R_3SnOH + HJ$$

$$R_3SnOH \rightarrow (R_3Sn)_2O + H_2O$$

führt. Durch ein Äquivalent Wasser werden dabei zwei Äquivalente des Organozinniodids in ein katalytisch inaktives Organozinnoxid umgewandelt.

Schon 0,1 % Wasser in dem für die Isomerisierung eingesetzten Vinyloxiran birgt die Gefahr wirtschaftlich nicht mehr tragbarer Verluste an Organozinnverbindung in sich.

Als Lewis-Säuren für die Isomerisierung von Vinyloxiran zu 2,5-Dihydrofuran eignen sich auch Zinkhalogenide (EP 412 366 und WO 93/10111), die in technischen Mengen preisgünstig verfügbar sind. Bei Anwendung des in US 5 238 889 beschriebenen Verfahrens zur Abscheidung von oligomeren Nebenprodukten durch Zugabe eines unpolaren Lösungsmittels zu einer gebrauchten Katalysatormischung aus einem Oniumiodid und Zinkchlorid, -bromid oder -iodid war jedoch zu erwarten, daß sich das Zinkhalogenid wegen seines Salzcharakters in der polaren Oligomerenphase anreichert, insbesondere, wenn man bedenkt, daß Zinkhalogenide sich in Kohlenwasserstoffen nicht lösen. Überraschenderweise ist genau das Gegenteil der Fall:
Das Zinksalz wird zusammen mit dem Oniumiodid in der unpolaren Phase angereichert, so daß die Oligomeren ohne nennenswerte Katalysatorverluste abgereichert werden können.

Demgemäß ist Gegenstand der Erfindung ein Verfahren zur Abtrennung eines Katalysators aus einem Gemisch mit Oligomeren des Vinyloxirans, wie es bei der Isomerisierung zu 2,5-Dihydrofuran entsteht und aus dem praktisch alle Leichtsieder abdestilliert worden sind, wobei man das Gemisch der Oligomeren mit einem Katalysator, bestehend im wesentlichen aus

a) einem Oniumiodid,
b) einer Lewis-Säure ausgewählt aus dem Chlorid, Bromid oder Iodid des Kobalts, Bismuths, Zinns oder Zinks und gegebenenfalls

c) einem Donorliganden

(1) mit einem Kohlenwasserstoff oder Chlorkohlenwasserstoff mit 5 bis 14 Kohlenstoffatomen intensiv vermischt,
(2) aus den sich bildenden 2 Phasen die den Katalysator enthaltende Phase abtrennt und
(3) den Katalysator durch Abdestillieren des Lösungsmittels gewinnt.

Das Abtrennungsverfahren kann kontinuierlich oder diskontinuierlich durchgeführt und in das Isomerisierungs-Verfahren integriert werden. Dabei wird die Isomerisierung des Vinyloxirans wie in der US 5,238,889 beschrieben durchgeführt. Auf diese Patentschrift wird deshalb, auch im Hinblick auf die Beschreibung der Oniumhalogenide, ausdrücklich Bezug genommen und deren Angaben - ausgenommen die andere Art der Lewis-Säure (b) - sollen als in die vorliegende Beschreibung integriert gelten.
Demgemäß schließt das Kohlenwasserstoff- oder Chlorkohlenwasserstoff-Extraktionsmittel mit 5 bis 14 C-Atomen, bevorzugt 8 bis 12 C-Atomen, geradkettige oder verzweigte, acyclische oder cyclische Verbindungen ein. Spezifische Beispiele von acyclischen Extraktionsmitteln sind Pentan, Hexan, Heptan, Octan, Nonan, Decan, gemischte Decane, gemischte Heptane, gemischte Octane oder Isooctan.
Beispiele von cyclischen Kohlenwasserstoff-Extraktionsmitteln sind vor allem Cycloalkane mit 6 bis 12 C-Atomen. Im einzelnen sind zu nennen: Cyclohexan, Cyclooctan und Cyclododekan, sowie Dekalin.
Als Chlorkohlenwasserstoffe kommen z.B. vorzugsweise monochlorierte Verbindungen mit 8 bis 12 C-Atomen in Betracht.
Als Oniumverbindungen (a) kommen alle die in US 5,238,889 definierten Verbindungen, insbesondere jedoch solche der Formel

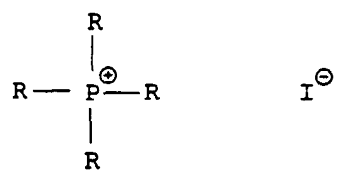

in Betracht, in der R einen Kohlenwasserstoffrest mit 4 bis 8 C-Atomen bedeutet.
Als Katalysatorbestandteile (a) kommen über die in US 5,238,889 beschriebenen Oniumverbindungen, ausgewählt aus Tetra-alkyl- und/ oder aryl-ammoniumiodiden und Tetra-alkyl- und/oder aryl-phosphoniumiodiden mit 16 bis 72 C-Atomen und den dort genannten Einzelverbindungen hinaus Phosphazenium- oder Phosphazaniumiodide mit 60 bis 144 C-Atomen in Betracht, soweit diese in den erfindungsgemäß zu verwendenden Extraktionsmitteln, gegebenenfalls auch erst bei höherer Temperatur, zu mindestens 10 % löslich sind. Insbesondere kommen Phosphazeniumiodide in Betracht. Diese Phosphazeniumiodide werden ausgewählt aus den in DE-A 44 24 219(O.Z. 45023) definierten Verbindungen der Formel

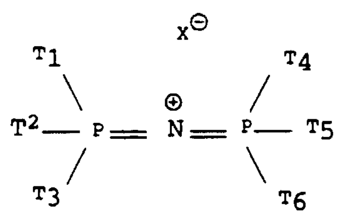

in der die Reste $T^1$ bis $T^6$ die in DE-A 44 24 219(O.Z. 45023) angegebenen Bedeutungen haben und $X^{\ominus}$ ein Gegenanion bedeutet.
Insbesondere kommen solche Verbindungen in Betracht, bei denen $T^1$ bis $T^6$ Phenylreste bedeuten, die durch insgesamt 6 bis 18 niedermolekulare Alkylreste substituiert sind.
Als Lewis-Säuren (b) werden erfindungsgemäß die Chloride, Bromide oder Iodide des Kobalts, Zinns, Bismuths oder Zinks oder Mischungen dieser Verbindungen eingesetzt. Davon ist Zinkiodid bevorzugt.

Für eine gute Selektivität der Isomerisierung von Vinyoxiran zu 2,5-Dihydrofuran wird die Lewis-Aktivität z.B. des Zinkhalogenids durch einen schwach basischen Donorliganden (c) abgeschwächt. Geeignete Verbindungen sind die in DE-A 44 24 219 (O.Z. 45023) beschriebenen Verbindungen. Insbesondere sind tertiäre Amide wie N,N-Dimethyloctancarbonsäureamid, N,N-Dimethylbuttersäureamid, Lactame wie N-Cyclohexylpyrrolidon, N-Octylpyrrolidon, N-Octylcaprolactam oder Harnstoffe wie Tetrabutylharnstoff, Tetraoctylharnstoff, N,N-Dioctylethylen- oder -propylenharnstoff zu nennen, wobei die Amide, Lactame oder Harnstoffe 16 bis 54 C-Atome enthalten sollen.

Als Donorliganden eignen sich auch besonders Trialkyl- oder Triarylphosphanoxide, wie Tributylphosphanoxid, Trioctylphosphanoxid oder Triphenylphosphanoxid sowie Phosphorsäuretriamide, wie Hexabutyl- oder Hexaoctylphosphorsäuretriamid.

Da, bezogen auf zu isomerisierendes Vinyloxiran, die Katalysatorkomponente (a) in der Regel in Mengen von 0,01 bis 5 Gew.-%, vorzugsweise von 0,5 bis 1 Gew.-% und die Katalysatorkomponente (b) in Mengen von 0,0001 bis 0,5 Gew.-%, vorzugsweise von 0,001 bis 0,2 Gew.-% eingesetzt wird, berägt das Mengenverhältnis der Komponente (a) zu (b) im Gemisch 1 zu 0,001 bis 1 zu 0,1, vorzugsweise 1 zu 0,01 bis 1 zu 0,1. Der Anteil des Donorliganden (c) am Katalysatorgemisch beträgt in der Regel 0,01 bis 30 Gew.-%, vorzugsweise 0,1 bis 10 Gew.-%.

Der Anteil des gesamten Katalysatorgemisches am erfindungsgemäß zu trennenden Gemisch (d.i. nach Entfernung praktisch aller Leichtsieder wie Dihydrofuran, Vinyloxiran und Cotonaldehyd) mit den Oligomeren hängt von der Menge des in der Isomerisierung eingesetzten Katalysatorgemisches und dem Umfang der Ausschleusung der Oligomeren ab. Er beträgt in der Regel 20 bis 90 vorzugsweise 50 bis 80 Gew.-%, bezogen auf das gesamte Katalysator/Oligomeren-Gemisch.

Das erfindungsgemäße Katalysator-Wiedergewinnungsverfahren kann ansatzweise, halbkontinuierlich oder kontinuierlich ausgeführt werden. Bei der ansatzweisen Verfahrensführung werden die flüchtigen Komponenten des Isomerisierungs-Reaktionsgemisches abdestilliert, zum Rückstand das Extraktions-Lösemittel zugesetzt, die Komponenten intensiv vermischt und nach Phasentrennung das Katalysatorgemisch durch Abdestillieren des Extraktionsmittels isoliert. Dabei sollten alle, oder praktisch alle flüchtigen Komponenten aus dem Katalysator/Oligomeren-Gemisch entfernt sein, weil sonst ein Teil der Oligomeren in dem Extraktionsmittel solubilisiert wird. Die Menge der flüchtigen Komponenten des Katalysatorsystems beträgt in der Regel 5 bis 30 Gew.-% der zu extrahierenden Katalysator/Oligomeren-Mischung. Die Extraktion der Oligomerenphase kann, falls erforderlich, wiederholt und die Extrakte zur Aufarbeitung durch Eindampfen vereinigt werden. Man erhält das Katalysatorsystem als geschmolzene Flüssigkeit, die ohne weitere Reinigung in den Isomerisierungsprozeß zurückgeführt werden kann. Geringe Mengen Oligomer, die noch im Katalysatorsystem verbleiben, stören bei der Wiederverwendung in der Isomerisierung nicht.

Das Oligomere kann als Nebenprodukt isoliert werden oder verworfen werden. Aus dem geschmolzenen Katalysatorgemisch können auch die Katalysatorbestandteile durch Kristallisation gewonnen werden, doch ist die direkte Rückführung bevorzugt.

Das erfindungsgemäße Extraktionsverfahren wird in der Regel bei leicht erhöhter Temperatur durchgeführt, um eine gute Löslichkeit des Katalysators im Lösemittel und eine verringerte Viskosität der Oligomeren zu gewährleisten. Man wählt aber zweckmäßig eine Temperatur unterhalb des Siedepunkts des Lösemittels, um bei atmosphärischem Druck arbeiten zu können. Dies bedeutet, daß die Extraktion üblicherweise bei Temperaturen von 40 bis 125°C ausgeführt wird.

In der Regel wird jedoch die Extraktion halbkontinuierlich oder vorzugsweise kontinuierlich nach Standardverfahren des Standes der Technik, z.B. gemäß den Angaben in T.C.Lo, M.H.I. Baird, C. Hanson, Handbook of Solvent Extraktion, Reprint Edition, Krieger Publishing Company, Malabar, Fla. U.S.A. 1991, ausgeführt. Typische Gegenstrom Extraktionssysteme sind beispielsweise Mixer/ Settler, Siebbodenkolonnen, gerührte Kolonnen wie Kuhni oder Rotating-Disk-Kolonnen oder Kolonnen mit mechanisch bewegten Böden. Bei der kontinuierlichen Verfahrensführung wird ständig ein Teil der Katalysator/Oligomeren-Mischung aus dem Reaktor ausgeschleust und die flüchtigen Bestandteile kontinuierlich abdestilliert. Die konzentrierte Mischung wird dann kontinuierlich einer mehrstufigen Extraktionsvorrichtung im Gegenstrom zum Extraktionsmittel zugeführt. Das Katalysatorgemisch wird dann aus dem Extrakt durch Verdampfen erhalten.

Die Menge des zur Extraktion verwendeten Lösemittels hängt von der Art des Katalysatorsystems, dessen Anteil in Oligomerengemisch und der Art des Lösemittels sowie der Art und Weise, wie die Extraktion ausgeführt wird, ab. Jedoch wird das Mengenverhältnis Lösemittel zu Katalysator/Oligomeren-Gemisch üblicherweise 10 : 1 bis 0,1 : 1 gewählt.

Beispiel 1 (Katalysatorabtrennung)

In einem ölbeheizten Rührkolben wird eine Mischung von 85,6 g Tri-n-octyl(n-octadecyl)phosphoniumiodid, 3,8 g Zinkiodid und 11,5 g N-Cyclohexylpyrrolidon auf 100°C unter Rühren in Stickstoffatmosphäre erhitzt. Man pumpt mit einer Mengen-gesteuerten Pumpe 1 840 g Vinyloxiran mit der Geschwindigkeit von 40 g/Std. gleichmäßig zu. Das

gebildete 2,5-Dihydrofuran wird zusammen mit Resten nicht umgesetzten Vinyloxirans und Spuren Crotanaldehyds laufend abdestilliert. Nach Ende des Zulaufs wird der Druck allmählich bis auf 10 mbar abgesenkt, um die Leichtsieder möglichst weitgehend abzudestillieren.

Insgesamt werden 1 766 g Destillat gewonnen, das zu 83,5 % aus 2,5-Dihydrofuran, zu 15 % aus Vinyloxiran und zu 1,52 % aus Crotonaldehyd besteht. Die Selektivität an 2,5-Dihydrofuran ist 93,6 % bei 85,6 % Umsatz.

Der bei Raumtemperatur dickflüssige Destillationsrückstand wird mit 350 ml n-Octan vermischt und unter Rühren auf 80°C erhitzt. Nach Abstellen des Rührens wird die Unterphase abgetrennt, mit weiteren 250 ml n-Octan bei 80°C durchmischt und anschließend die Unterphase wieder abgetrennt.

Nach dem Abdampfen der Unterphase am Rotationsverdampfer bei 80°C/14 mbar verbleiben 40 g Destilltionsrückstand. Dieser Rückstand enthält 1,3 % Iod und 0,03 % Zink. Aus diesen analytischen Daten ist zu schließen, daß 97 % des Iods und 98,5 % des Zinks in den beiden Octanphasen verblieben sind.

Nach Vereinigung der beiden Octanphasen werden diese bei 80°C Badtemperatur und 14 mbar am Rotationsverdampfer abgedampft, wobei 135 g Rückstand verbleiben, der bei Raumtemperatur erstarrt.

Beispiel Ia (Katalysatorrückführung)

Der Abdampfrückstand aus den Octanphasen von Beispiel 1 wird auf 125°C erhitzt. Unter Rühren pumpt man 800 g Vinyloxiran mit der Geschwindigkeit von 35 g/Std. kontinuierlich zu und destilliert 2,5-Dihydrofuran zusammen mit nicht umgesetztem Vinyloxiran ab. Nach Ende des Zulaufs werden die Leichtsieder durch Absenken des Drucks bis auf 10 mbar weitgehend abdestilliert. Man erhält 747,2 g Destillat mit der Zusammensetzung 82 % 2,5-Dihydrofuran, 16,5 Vinyloxiran und 1,5 % Crotanaldehyd.

Beispiel 2

Eine Mischung von 42,8 g Tri-n-octyl(-n-octadocyl)phosphoniumiodid, 1,92 g Zinkiodid und 33 g Tri-n-octylphosphanoxid wird unter Rühren auf 125°C erhitzt. 1 442 g Vinyloxiran werden mit einer Geschwindigkeit von 20 g/Std. zugepumpt. Das durch Isomerisierung gebildete 2,5-Dihydrofuran wird zusammen mit nicht umgesetztem Vinyloxiran und Spuren Crotanaldehyds laufend abdestilliert. Nach Ende des Zulaufs wird der Druck allmählich bis auf 10 mbar abgesenkt, um die Leichtsieder möglichst weitgehend abzudestillieren.

Insgesamt werden 1 370 g Destillat erhalten mit der Zusammensetzung 85 % 2,5-Dihydrofuran, 1,1 % Crotanaldehyd und 13,9 % Vinyloxiran. Daraus berechnet sich eine Selektivität von 93 % bei 86,8 % Umsatz.

Der Destillationsrückstand wird wie in Beispiel 1 beschrieben bei 80°C nacheinander mit 250 und 172 ml n-Octan extrahiert. Nach Verdampfen von Octanresten bei 80°C/16 mbar verbleiben 22 g eines Rückstands aus oligomerem Vinyloxiran, der 0,70 % Iod und 0,015 % Zink enthält. Aus diesen Daten ist zu schließen, daß 98 % des Iods und 99,2 % des Zinks in den beiden Octanphasen verblieben sind.

Die abgedampften Octanphasen führen zu einem Isomerisierungskatalysator, der unter den oben beschriebenen Bedingungen unveränderte Aktivität und Selektivität zeigt und durch weitere Reinigung in die Isomerisierungsreaktion zurückgeführt werden kann.

**Patentansprüche**

1. Verfahren zur Abtrennung eines Katalysators aus einem Gemisch mit Oligomeren des Vinyloxirans, wie es bei der Isomerisierung zu 2,5-Dihydrofuran entsteht und aus dem praktisch alle Leichtsieder abdestilliert worden sind, dadurch gekennzeichnet, daß man das Gemisch der Oligomeren mit einem Katalysator, bestehend im wesentlichen aus

    a) einem Oniumiodid,
    b) einer Lewis-Säure ausgewählt aus dem Chlorid, Bromid oder Iodid des Kobalts, Bismuths, Zinns oder Zinks und gegebenenfalls
    c) einem Donorliganden,

        (1) mit einem Kohlenwasserstoff oder Chlorkohlenwasserstoff mit 5 bis 14 Kohlenstoffatomen intensiv vermischt,
        (2) aus den sich bildenden 2 Phasen die den Katalysator enthaltende Phase abtrennt und
        (3) den Katalysator durch Abdestillieren des Lösungsmittels gewinnt.

2. Verfahren nach Anspruch 1, dadurch gekennzeichnet, daß man das zu trennende Gemisch mit einem Alkan mit

5 bis 14 C-Atomen extrahiert.

**3.** Verfahren nach Anspruch 1, dadurch gekennzeichnet, daß man das Gemisch mit einem Katalysator, bestehend im wesentlichen aus einem Phosphoniumiodid, Zinkiodid und einem geradkettigen oder cyclischen N-substituierten Amid oder einem Trialkylphosphanoxid extrahiert.

**4.** Verfahren nach Anspruch 1, dadurch gekennzeichnet, daß man mit einem Kohlenwasserstoff mit 8 bis 12 Kohlenstoffatomen extrahiert.

## Claims

**1.** A process for separating a catalyst from a mixture with oligomers of vinyloxirane, as is formed in the isomerization to 2,5-dihydrofuran and from which virtually all low boilers have been distilled off, wherein the mixture of oligomers with a catalyst consisting essentially of

a) an onium iodide,
b) a Lewis acid selected from the group consisting of the chlorides, bromides and iodides of cobalt, bismuth, tin and zinc, and possibly
c) a donor ligand,

(1) is intensively mixed with a hydrocarbon or chlorinated hydrocarbon having from 5 to 14 carbon atoms,
(2) the catalyst-containing phase is separated from the 2 phases which form and
(3) the catalyst is isolated by distilling off the solvent.

**2.** A process as claimed in claim 1, wherein the mixture to be separated is extracted with an alkane having from 5 to 14 carbon atoms.

**3.** A process as claimed in claim 1, wherein the mixture with a catalyst consisting essentially of a phosphonium iodide, zinc iodide and a straight-chain or cyclic N-substituted amide or a trialkylphosphine oxide is extracted.

**4.** A process as claimed in claim 1, wherein the extraction is carried out using a hydrocarbon having from 8 to 12 carbon atoms.

## Revendications

**1.** Procédé de séparation d'un catalyseur à partir d'un mélange avec des oligomères de vinyloxirane, tel qu'obtenu lors de l'isomérisation en 2,5-dihydrofurane et duquel pratiquement tous les composés à bas points d'ébullition ont été éliminés par distillation, caractérisé en ce que le mélange des oligomères et du catalyseur, constitué essentiellement :

a) d'un iodure d'onium,
b) d'un acide de Lewis choisi parmi les chlorures, bromures ou iodures du cobalt, du bismuth, de l'étain ou du zinc et éventuellement,
c) d'un ligand donneur,

(1) est mélangé de façon énergique avec un hydrocarbure ou un hydrocarbure chloré ayant 5 à 14 atomes de carbone,
(2) on sépare des deux phases formées, celle contenant le catalyseur et
(3) on récupère le catalyseur en éliminant le solvant par distillation.

**2.** Procédé selon la revendication 1, caractérisé en ce que l'on extrait le mélange à séparer avec un alcane ayant 5 à 14 atomes de carbone.

**3.** Procédé selon la revendication 1, caractérisé en ce que l'on extrait le mélange avec un catalyseur constitué essentiellement d'un iodure de phosphonium, d'un iodure de zinc et d'un amide N-substitué à chaîne droite ou cyclique ou d'un trialkylphosphanoxyde.

**4.** Procédé selon la revendication 1, caractérisé en ce que l'on extrait avec un hydrocarbure ayant 8 à 12 atomes de carbone.